# EUROPEAN PATENT APPLICATION

(11) **EP 4 667 001 A2**
(43) Date of publication of application: **24.12.2025**
(21) Application number: 25206511.5
(22) Date of filing: 19.04.2022
(51) Int. Cl.: A61K 9/16

(54) **FORMULATIONS OF CANNABINOIDS**

(30) Priority: 16.04.2021 EP 21168880; 11.05.2021 EP 21173344
(62) Divisional of application: 22723420.0
(71) Applicant: ADD Advanced Drug Delivery Technologies Ltd., 4133 Pratteln (CH)
(72) Inventor: NOWAK, Reinhard, 79589 Binzen (DE); NOWAK, Mirko, 79540 Lörrach (DE); NOWAK, Jay Jesko, 79540 Lörrach (DE); PÖLLINGER, Norbert, 79379 Müllheim (DE)
(74) Representative: Breuer, Markus

(57) **Abstract**

Pharmaceutical formulations of cannabinoids, in particular of cannabidiol, are provided. The formulations are in the form of a solid dispersion, wherein the solid dispersion comprises in admixture a cannabinoid, an amphiphilic block copolymer as a solubilizer, a water-soluble film former and an antioxidant.

## Description

### Field of the Invention

The present invention relates to formulations of cannabinoids, in particular of cannabidiol, as well as methods for preparing such formulations. The invention provides solid formulations for oral administration of cannabinoids, in particular of cannabidiol.

### Background of the Invention

Cannabinoids and in particular cannabidiol (CBD) have been considered as drugs. There is evidence that cannabinoids can be beneficial for treating a number of clinical conditions, including pain, inflammation, epilepsy, sleep disorders, indication of multiple sclerosis, anorexia, and schizophrenia (N. Bruni et al., Cannabinoid Delivery Systems for Pain and Inflammation Treatment. Molecules 2018, 23, 2478).

While the use of cannabinoids in various indications has been suggested, so far only limited applications received market authorisation.

Cannabinoids, in particular cannabidiol, are difficult to formulate due to their highly lipophilic nature. Low and variable bioavailability of cannabinoids, in particular upon oral administration, hampers effective clinical use of these compounds.

In fact, cannabinoids are highly lipophilic molecules (log P 6-7) with very low water solubility (2-10 µg / ml). The log P is the decimal logarithm of the n-octanol/water partition coefficient. The partition coefficient can be determined experimentally. Values typically refer to room temperature (25°C). The partition coefficient can also be roughly calculated from the molecular structure.

In addition to poor solubility, cannabinoids, in particular CBD, are subject to high first-pass metabolism, which further contributes to poor systemic availability after oral administration.

Various formulations of cannabinoids have been suggested.

Due to the high lipophilicity of cannabinoids, salt formation (*i.e.* pH adjustment), cosolvency (*e.g.* ethanol, propylene glycol, PEG400), micellization (*e.g.* Polysorbate 80, Cremophor-ELP), emulsification including micro and nano emulsification, complexation (*e.g.* cyclodextrins) and encapsulation in lipid-based formulations (*e.g.* liposomes) are among the formulation strategies considered in the prior art. Nanoparticle systems have also been proposed (N. Bruni *et al., loc. cit*.).

Various solid oral dosage forms have been proposed in the patent literature, for example in WO 2008/024490 A2 and in WO 2018/035030 A1. These documents do not contain data on release behaviour, so the practical suitability of the proposed forms for the administration of cannabinoids remains unclear.

WO 2015/065179 A1 describes compressed tablets which, in addition to cannabidiol, contain lactose and sucrose fatty acid monoesters.

Dronabinol (Δ9-THC) is marketed in the form of capsules (Marinol^{®}) and as an oral solution (Syndros^{®}). The Marinol^{®} capsules are soft gelatine capsules containing the active ingredient in sesame oil.

The drug product Sativex^{®} containing nabiximols is a mouth spray that is sprayed onto the inside of the cheek.

Self-emulsifying drug delivery systems (SEDDS) which are mixtures of oils, surfactants and optionally contain hydrophilic solvents have also gained interest in an approach to improve the oral bioavailability of certain cannabinoids (K. Knaub *et al.* (2019). A Novel Self-Emulsifying Drug Delivery System (SEDDS) Based on VESIsorb^{®} Formulation Technology Improving the Oral Bioavailability of Cannabidiol in Healthy Subjects. Molecules, 24(16), 2967). Upon contact with an aqueous phase, such as gastric or intestinal fluids, SEDDS spontaneously emulsify under conditions of gentle agitation.

VESIsorb^{®}, a self-emulsifying drug delivery formulation technology developed by Vesifact AG (Baar, Switzerland) has shown increased oral bioavailability of certain lipophilic molecules.

The preparation Epidiolex^{®} recently approved by the US-FDA as an orphan drug for the treatment of certain forms of epilepsy is provided in the form of an oral solution that in addition to the active ingredient cannabidiol contains the excipients absolute ethanol, sesame oil, strawberry aroma and sucralose.

Notwithstanding all these proposals, there is still a need for improved dosage forms for cannabinoids, such as cannabidiol, in particular for solid oral dosage forms.

There is in particular a need for solid oral dosage forms showing high bioavailability of the cannabinoid and/or a reduced food effect and/or reduced variability of pharmacokinetic parameters.

### Summary of the Invention

The present invention provides a pharmaceutical formulation in the form of a solid dispersion, wherein the solid dispersion comprises in admixture a cannabinoid, an amphiphilic block copolymer as a solubilizer and a water-soluble film former.

The amphiphilic block copolymer is preferably a block copolymer containing at least one polyoxyethylene block and at least one polyoxypropylene block, such as a poloxamer.

The water-soluble film former is preferably polyvinylpyrrolidone or hydroxypropylmethyl cellulose.

The above components are present in a weight ratio cannabinoid: amphiphilic block copolymer : water soluble film former of typically 1 : 0.11 - 0.41 : 0.03 - 0.33, preferably 1 : 0.16 - 0.36 : 0.08 - 0.28, more preferably 1 : 0.21 - 0.31 : 0.13 - 0.23.

The cannabinoid is in particular cannabidiol (2-[(1R,6R)-3-methyl-6-(1-methylethenyl)-2-cyclohexen-1-yl]-5-pentyl-1,3-benzenediol).

A formulation according to the present invention may consist of a cannabinoid, an amphiphilic block copolymer as a solubilizer, a water-soluble film former, an antioxidant, a diluent and a moisture adsorbent and not more than 10 % by weight of other components, preferably not more than 5 % by weight of other components, in particular not more than 2 % of other components, relative to all components of the formulation.

A formulation according to the present invention preferably contains an antioxidant, in particular ascorbyl palmitate.

The present invention provides formulations which achieve a high bioavailability of the cannabinoid. The bioavailability can be assessed by the AUC over an interval of 24 hours. Determination is, for instance, after a light meal of 350 - 600 kcal taken within 30 min prior to each administration within the 24 hours interval of determining the AUC.

The invention also provides a method for preparing a cannabinoid containing formulation which comprises the steps of (i) preparing a liquid composition comprising the cannabinoid, the amphiphilic block copolymer and a solvent capable of at least partially dissolving the cannabinoid and the amphiphilic block copolymer; (ii) introducing the liquid composition into a fluid bed granulator; (iii) removing solvent to obtain a solid dispersion in particulate form; and (iv) recovering the solid dispersion in particulate form from the fluid bed granulator.

Further objectives and their solution can be concluded from the detailed description of the invention below.

### Detailed Description of the Invention

### Active Ingredients

Cannabinoids are a heterogeneous group of pharmacologically active substances that have an affinity for the so-called cannabinoid receptors. The cannabinoids include, for example, tetrahydrocannabinol (THC) and the non-psychoactive cannabidiol (CBD).

Cannabinoids can be both phytocannabinoids and synthetic cannabinoids.

Phytocannabinoids are a group of about 70 terpenophenolic compounds (V.R. Preedy (ed.), Handbook of Cannabis and Related Pathologies (1997)). These compounds typically contain a monoterpene residue that is attached to a phenolic ring and has a C₃-C₅ alkyl chain that is in the meta position to the phenolic hydroxyl group.

A preferred group of cannabinoids are tetrahydrocannabinols with the following general formula (1): 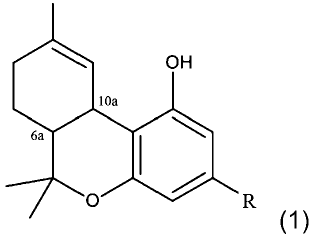 wherein R is selected from among C₁-C₂₀-alkyl, C₂-C₂₀-alkenyl or C₂-C₂₀-alkynyl, and optionally has one or more substituents.

In a further preferred group of compounds of the above general formula (1), R is selected from among C₁-C₁₀-alkyl or C₂-C₁₀-alkenyl, and optionally has one or more substituents.

In particular, in formula (1) R is an alkyl radical with the formula C₅H₁₁.

Compounds of general formula (1) can be present in the form of stereoisomers. The centres 6a and 10a preferably each have the R configuration.

The tetrahydrocannabinol is in particular Δ9-THC with the chemical name (6aR,10aR)-6,6,9-trimethyl-3-pentyl-6a, 7,8,10a-tetrahydro-6H-benzo[c]chromene-1-ol. The structure is reflected by the following formula (2):

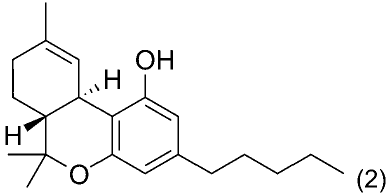

Another preferred group of cannabinoids are cannabidiols with the following general formula (3): 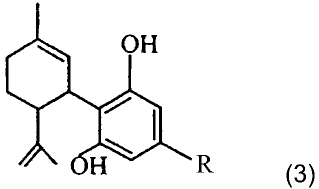 wherein R is selected from among C₁-C₂₀-alkyl, C₂-C₂₀-alkenyl or C₂-C₂₀-alkynyl, and optionally has one or more substituents.

In a further preferred group of compounds having the general formula (3) above, R is selected from among C₁-C₁₀-alkyl or C₂-C₁₀-alkenyl, and optionally has one or more substituents.

In particular, R in formula (3) is an alkyl radical with the formula C₅H₁₁.

The cannabidiol is in particular 2-[(1R,6R)-3-methyl-6-(1-methylethenyl)-2-cyclohexen-1-yl]-5-pentyl-1,3-benzenediol. In the present specification, if the term cannabidiol or its abbreviation CBD is used, this particular compound is meant, unless stated otherwise.

CBD is a major constituent of *Cannabis sp.* - besides the psychotropic Δ9-THC. The psychotropic effect of THC is mediated by the cannabinoid receptor CB1 that is mainly expressed on neurons. In contrast to THC, CBD is a peripherally and centrally acting compound without psychotropic activity.

According to the invention, a combination of Δ9-THC ((6aR, 10aR)-6,6,9-trimethyl-3-pentyl-6a,7,8,10a-tetrahydro-6H-benzo[c]chromen-1-ol) and CBD (2-[(1R,6R)-3-methyl-6-(1-methylethenyl)-2-cyclohexen-1-yl]-5-pentyl-1,3-benzenediol) can be used.

Another preferred group of cannabinoids are cannabinols with the following general formula (4): 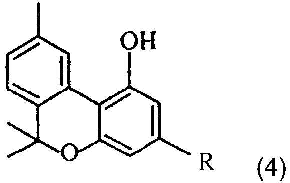 wherein R is selected from among C₁-C₂₀-alkyl, C₂-C₂₀-alkenyl or C₂-C₂₀-alkynyl, and optionally has one or more substituents.

In a further preferred group of compounds having the general formula (4) above, R is selected from among C₁-C₁₀-alkyl or C₂-C₁₀-alkenyl, and optionally has one or more substituents.

In particular, in formula (4) R is an alkyl radical having the formula C₅H₁₁.

The cannabinol is especially 6,6,9-trimethyl-3-pentyl-6*H*-dibenzo[*b,d*]pyran-1-ol.

According to the invention, cannabinoids or cannabinoid mixtures of hemp extracts can also be used.

For example, Nabiximols is a plant extract mixture used as a drug of the leaves and flowers of the hemp plant (Cannabis sativa L.) with standardized contents of tetrahydrocannabinol (THC) and cannabidiol (CBD).

Synthetic cannabinoids can also be used.

These include 3-(1,1-dimethylheptyl)-6,6a,7,8,10,10a-hexahydro-1-hydroxy-6,6-dimethyl-9*H*-dibenzo[*b,d*]pyran-9-one. This compound contains two stereogenic centres. The drug nabilone is a 1:1 mixture (racemate) of the (6aR,10aR) form and the (6aS,10aS) form. Nabilone is a preferred cannabinoid according to the invention.

Another example of a synthetic cannabinoid is JWH-018 (1-naphthyl-(1-pentylindol-3-yl)methanone).

The use of cannabinoids, in particular of cannabidiol, is based on their pharmacodynamic properties. Cannabinoid receptors include CB1, which is predominantly expressed in the brain, and CB2, which is primarily found on the cells of the immune system. The fact that both CB1 and CB2 receptors have been found on immune cells suggests that cannabinoids play an important role in the regulation of the immune system. Independent of this finding, several studies show that cannabinoids downregulate cytokine and chemokine production and, in some models, upregulate T-regulatory cells (Tregs) as a mechanism to suppress inflammatory responses. The endocannabinoid system is also involved in immunoregulation.

### Galenics

The present invention provides a pharmaceutical formulation which is a solid dispersion comprising a cannabinoid, in particular cannabidiol. As further detailed below, solid dosage forms for oral administration showing satisfactory bioavailability can be obtained in this way. Dosage forms according to the present invention also show a reduced food effect.

A highly lipophilic cannabinoid, like the almost water insoluble CBD, is combined with an amphiphilic block copolymer and with a water-soluble film former to form a solid dispersion. Incorporating a combination of a solubilizer and a water-soluble film former into the formulation allows adjusting the release rate of the cannabinoid.

The solid dispersion comprising a cannabinoid, in particular cannabidiol, an amphiphilic block copolymer and a water-soluble film former leads to the formation of micelles upon contact with water or other aqueous media, such as gastrointestinal fluids. The micelles are essentially formed from the drug substance, surrounded by the solubilizing excipients (see Fig. 1).

One aspect of the invention is accordingly a micellar composition comprising an aqueous phase in which micelles are dispersed, which micelles comprise a cannabinoid, in particular cannabidiol, and solubilizing excipients, in particular the amphiphilic block copolymer and the water-soluble film former.

The amphiphilic block copolymer present in the formulations of the present invention acts as a solubilizer. The reference to an amphiphilic block copolymer includes the possibility that more than one such copolymer is present.

The cannabinoid and the amphiphilic block copolymer are present in a weight ratio cannabinoid: amphiphilic block copolymer of typically 1 : 0.11 - 0.41, preferably 1 : 0.16 - 0.36, more preferably 1 : 0.21 - 0.31.

The amphiphilic block copolymers are solid at ambient temperature.

They have surfactant properties and, if used in appropriate concentration ranges in aqueous media, in particular water, can form micellar solutions.

In particular block copolymers containing at least one polyoxyethylene block and at least one polyoxypropylene block can be used.

Preferred block copolymers are poloxamers. Poloxamers are block copolymers whose molecular weights range from 1,100 to over 14,000. Different poloxamers differ only in the relative amounts of propylene and ethylene oxides added during manufacture.

Poloxamers have the following general formula:

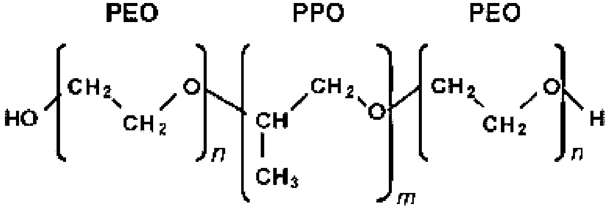

In this general formula, n designates the number of polyoxyethylene units, m designates the number of polyoxypropylene units.

In one embodiment, the solubilizer is Poloxamer 188 (Kolliphor P188; former brand name Lutrol F 68) / BASF; CAS No.: 9003-11-6).

Kolliphor P188 is a polyoxyethylene-polyoxypropylene block copolymer of the above general formula wherein n is approximately 79 and m is approximately 28.

Kolliphor P188 is available as a white to slightly yellowish waxy substance in the form of micropearls having a melting point of 52 - 57°C. It meets the requirements of Ph.Eur., USP / NF for Poloxamer 188.

As a further excipient, the formulations of the present invention contain a water-soluble film former. The reference to a water-soluble film former again includes the possibility that a combination of two or more such film formers is used.

The cannabinoid and the water soluble film former are present in a weight ratio cannabinoid : water soluble film former of typically 1 : 0.03 - 0.33, preferably 1 : 0.08 - 0.28, more preferably 1 : 0.13 - 0.23.

The water-soluble film former acts as a polymeric binder and additional solubilizer in the present formulation.

Examples of suitable water-soluble film formers are methyl cellulose (MC), hydroxypropyl methyl cellulose (HPMC), hydroxypropyl cellulose (HPC), hydroxyethyl cellulose (HEC), sodium carboxymethyl cellulose (Na-CMC) and polyvinyl pyrrolidone (PVP).

A preferred film former is PVP, in particular PVP K30 (such as Kollidon^{®} 30).

Another preferred film former is hydroxypropylmethyl cellulose (HPMC), in particular low-viscosity HPMC, such as HPMC with a viscosity of a 2% (w/w) aqueous solution at 20°C of 6 mPa·s or less.

The above discussed components are present in a weight ratio cannabinoid (in particular cannabidiol) : amphiphilic block copolymer: water soluble film former (polyvinylpyrrolidone) of typically 1 : 0.11 - 0.41 : 0.03 - 0.33, preferably 1 : 0.16 - 0.36 : 0.08 - 0.28, more preferably 1 : 0.21 - 0.31 : 0.13 - 0.23.

It is in particular considered to further include an antioxidant or a combination of antioxidants to protect the cannabinoid, in particular cannabidiol, from oxidation.

Cannabinoids, in particular cannabidiol, are susceptible to oxidation. For instance, cannabidiol can be oxidized to monomeric and dimeric hydroxyquinones. The oxidation can lead to discoloration.

The oxidation can not only occur by molecular oxygen, but also by peroxides which may be introduced into the formulation by one or more of the excipients used.

Useful antioxidants which may be included into the formulation encompasses ascorbyl palmitate, alpha-tocopherol, butylhydroxytoluol (BHT, E321), butylhydroxyanisol (BHA, E320), ascorbic acid, and ethylenediaminetetraacetic acid (EDTA) sodium.

Ascorbyl palmitate is a preferred antioxidant. It can effectively suppress discoloration by oxidation.

The antioxidant is typically used in an amount of 0.5 to 2.5 wt%, preferably of 0.8 to 2 wt%, in particular 1.0 to 1.8 wt%, relative to the amount of the cannabinoid (in particular cannabidiol).

Other excipients may be present in addition.

In a preferred embodiment, the formulation contains in addition a diluent. Diluents (or fillers) as typically used in solid oral dosage forms can be employed. Such diluents are cellulosic materials, sugars, or inorganic salts. A preferred diluent is microcrystalline cellulose (such as Avicel^{®} PH 101). Another preferred diluent is mannitol (such as Pearlitol 160 C).

In formulations containing a diluent, there will typically be two phases, one phase comprising the active agent embedded in the polymeric excipients as detailed above and another phase comprising the diluent.

Active ingredient and diluent are typically present in a weight ratio cannabinoid (in particular cannabidiol) : diluent (in particular microcrystalline cellulose) of 1:0.5 - 2.7, preferably 1:0.9 - 2.3, in particular 1:1.3 - 1.9.

In a still further embodiments, silicon dioxide (such as Syloid^{®} 244 FP Silica) and/or colloidal silicon dioxide (such as Aerosil^{®} 200) are included in the formulation, in particular to serve as moisture adsorbents.

Active ingredient and total silicon dioxide components are typically present in a weight ratio cannabinoid (in particular cannabidiol) : total amount of all silicon dioxide components of 0.14 - 0.44, preferably 0.19 - 0.39, in particular 0.24 - 0.34.

While formulations according to the present invention are not limited to those containing the above discussed excipients, the formulations are preferably free or essentially free of triglycerides. Essentially free means that the formulation contains less than 5 % by weight, relative to all components, of triglycerides.

The solid dispersion is preferably free or essentially free of triglycerides. Essentially free means that the formulation contains less than 5 % by weight, relative to all components, of triglycerides.

Further, the solid dispersion is preferably free or essentially free of mono- and diglycerides. Essentially free means that the formulation contains less than 5 % by weight, relative to all components, of mono- and diglycerides.

Still further, the solid dispersion is preferably free or essentially free of fatty acids. Essentially free means that the formulation contains less than 5 % by weight, relative to all components, of fatty acids.

Preferably, the total amount of mono-, di- and triglycerides and fatty acids is less than 5 % by weight, relative to all components.

The present pharmaceutical formulations in the form of solid dispersions can be obtained by wet granulation techniques. The granulation can be carried out in a blender. Preferably, fluid bed granulation technology can be used.

According to the present invention, a method for preparing a cannabinoid containing formulation comprises the steps of (i) preparing a liquid composition comprising the cannabinoid, the amphiphilic block copolymer and a solvent capable of at least partially dissolving the cannabinoid and the amphiphilic block copolymer; (ii) introducing the liquid composition into a fluid bed granulator; (iii) removing solvent to obtain a solid dispersion in particulate form; and (iv) recovering the solid dispersion in particulate form from the fluid bed granulator.

According to the invention, the liquid composition comprising the cannabinoid, the amphiphilic block copolymer and the solvent preferably also comprises the water-soluble film former in at least partially dissolved form.

Further according to the invention, the liquid composition comprising the cannabinoid, the amphiphilic block copolymer and the solvent and optionally the water-soluble film former preferably also comprises the antioxidant in at least partially dissolved form.

The liquid composition may also comprise one or more further excipients. These can be present in any suitable form, for instance, in dissolved form or in dispersed form.

As an example, silicon dioxide can by present in the liquid composition in dispersed form.

The cannabidiol and the excipients are preferably present in the liquid compositions in the weight ratios as indicated herein for the pharmaceutical formulations.

The solvent used to prepare the liquid composition can be any solvent capable of at least partially dissolving the cannabinoid, the amphiphilic block copolymer and preferably also the water-soluble film former and/or the antioxidant.

A preferred solvent is ethanol comprising not more than 10% v/v water, such as ethanol comprising not more than 4% v/v water, for instance, ethanol 96% v/v.

As indicated above, the liquid composition is introduced into a fluid bed granulator. In a preferred embodiment, the liquid composition is sprayed into a fluid bed granulator already containing solid particles.

The solid particles contained in the granulator can comprise one or more excipients. In a preferred embodiment, the solid particles comprise a diluent, such as microcrystalline cellulose.

One or more additional excipient, such as colloidal silicon dioxide, can also be present.

The fluid bed granulator is operated so that solvent is removed and a solid dispersion in particulate form is obtained. For instance, an inlet air temperature of 45 ± 10°C can be chosen.

Solvent removal can be continued until a predetermined loss on drying (LOD) is reached. For instance, the product can be dried up to loss on drying of not more than 2.0%.

After drying the product is discharged and sieved.

The size of the granules obtained is not limited. Suitable sizes are in the range from 50 µm to 2000 µm, for example in the range from 100 µm to 1000 µm.

Formulations according to the present invention are preferably stable to discoloration. The color remains stable or changes only slightly to off-white upon storage for three months, preferably for six months and in particular for 12 months under long-term conditions (25°C/60% rh).

The granules represent a self-emulsifying solid dispersion. Upon combination with an aqueous medium a micellar solution can be obtained.

A formulation according to the invention, when subjected to an in vitro dissolution test in 0.1N HCl following the USP paddle method, releases at least 60 wt% of the cannabinoid within 60 minutes, preferably at least 90 wt% within 60 minutes, in particular at least 90 wt% within 45 minutes.

The solid dispersion granules can be filled into bottles, sachets or stick packs using commercial standard technology and equipment. The solid dispersion granules are to be sprinkled on food or dispersed in a liquid, *e.g.,* water.

A composition obtained by dispersing the solid dispersion granules in a liquid can be applied to patients being not able to swallow by means of a syringe through a gastric tube.

Depending on the final dosage strength per unit, the solid dispersion granules can also be filled into capsules which are feasible for swallowing (*e.g.* capsule size 2-1 for 25 mg/dose). Alternatively, for high dosed units, bigger capsules can be used as a primary packaging material for the granules. Such capsules are not for swallowing (*e.g.* capsule size up to 000 / sprinkle caps for 100-200 mg/dose). Rather, the solid dispersion granules are to be sprinkled on food or dispersed in a liquid, e.g., water.

Alternatively, the solid dispersion granules can also be processed into tablets. The solid dispersion granules are combined with one or more excipients, such as a disintegrant, a glidant, and/or a lubricant. The obtained mixture is then compressed into tablets.

In one embodiment, they are processed into orally dispersible tablets.

Solid dosage forms according to the present invention show increased bioavailability, reduced variability especially in the fasted state and better robustness towards food interaction compared to oil-based cannabidiol solutions.

Solid dosage forms according to the present invention containing cannabinoids are provided which show high bioavailability of the cannabinoid after oral administration.

For instance, according to the present invention, a steady state AUC of cannabidiol over 24 hours after repeated oral administration twice daily of a dose of 1500 mg cannabidiol in the form of a solid formulation of the present invention of about 9000 (h*ng/mL) is achieved.

The AUC of cannabidiol can, for instance, be within 9000 +/- 3000 (h*ng/mL).

The AUC as indicated above can be achieved after a light meal of 350 - 600 kcal taken within 30 min prior to each administration within the 24 hours interval of determining the AUC.

The steady state peak trough fluctuation (PTF) of cannabidiol over 24 hours after repeated administration twice daily of 1500 mg is about 250 %. The peak trough fluctuation can be determined as PTF = {[Cₘₐₓ - Cₘᵢₙ]/Cₐᵥ} x 100, wherein Cₘₐₓ, Cₘᵢₙ and the average Cₐᵥ represent steady state values for the 24 hours interval.

The steady state peak trough fluctuation (PTF) of cannabidiol can, for instance, be within 250 +/- 60 %.

The PTF as indicated above can be achieved after a light meal of 350 - 600 kcal taken within 30 min prior to each administration within the 24 hours interval of determining the peak and trough concentrations.

Further, according to the present invention, solid oral dosage forms are provided which achieve a bioavailability which is not inferior compared to that of an oily cannabidiol solution 100 mg/ml as a reference product. In case the cannabinoid is cannabidiol, a cannabidiol solution (DAC C-052 "Cannabidiol" / NRF 22.10 "Olige Cannabidiol-Lösung 100 mg/ml" ("Oily cannabidiol solution 100 mg/ml")) is the reference product. In this context, bioavailability means the systemic exposure as reflected by the AUC over a time period of 24 hours. The AUC is in particular achieved after a light meal of 350 - 600 kcal taken within 30 min prior to each administration within the 24 hours interval of determining the AUC.

### Aspects of the Invention

1. Pharmaceutical formulation in the form of a solid dispersion, wherein the solid dispersion comprises in admixture a cannabinoid, an amphiphilic block copolymer as a solubilizer and a water-soluble film former.
2. A formulation according to aspect 1, wherein the cannabinoid and the amphiphilic block copolymer are present in a weight ratio cannabinoid: amphiphilic block copolymer of 1 : 0.11 - 0.41, preferably 1 : 0.16 - 0.36, more preferably 1 : 0.21 - 0.31.
3. A formulation according to aspect 1 or 2, wherein the amphiphilic block copolymer is a block copolymer containing at least one polyoxyethylene block and at least one polyoxypropylene block.
4. A formulation according to aspect 3, wherein the amphiphilic block copolymer is a poloxamer, in particular poloxamer 188.
5. A formulation according to any of aspects 1 to 4, wherein the cannabinoid and the water soluble film former are present in a weight ratio cannabinoid : water soluble film former of 1 : 0.03 - 0.33, preferably 1 : 0.08 - 0.28, more preferably 1 : 0.13 - 0.23.
6. A formulation according to any of aspects 1 to 5, wherein the water soluble film former is polyvinylpyrrolidone, in particular PVP K-30.
7. A formulation according to any of aspects 1 to 5, wherein the water soluble film former is hydroxypropylmethyl cellulose.
8. A formulation according to any of aspects 1 to 7, wherein the components are present in a weight ratio cannabinoid: amphiphilic block copolymer : water soluble film former of 1 : 0.11 - 0.41 : 0.03 - 0.33, preferably 1 : 0.16 - 0.36 : 0.08 - 0.28, more preferably 1 : 0.21 - 0.31 : 0.13 - 0.23.
9. A formulation according to any of aspects 1 to 8, wherein the solid dispersion in addition comprises an antioxidant.
10. A formulation according to aspect 9, wherein the antioxidant is used in an amount of 0.5 to 2.5 wt%, preferably of 0.8 to 2 wt%, in particular 1.0 to 1.8 wt%, relative to the amount of the cannabinoid.
11. A formulation according to any of aspects 9 and 10, wherein the antioxidant is ascorbyl palmitate.
12. A formulation according to any of aspects 1 to 11, wherein the solid dispersion comprises a diluent.
13. A formulation according to aspect 12, wherein the cannabinoid and the diluent are present in a weight ratio cannabinoid: diluent of 1:0.5 - 2.7, preferably 1:0.9 - 2.3, in particular 1:1.3 - 1.9.
14. A formulation according to any of aspects 12 and 13, wherein the diluent is microcrystalline cellulose and/or mannitol.
15. A formulation according to any of aspects 1 to 14, wherein the solid dispersion comprises a moisture adsorbent.
16. A formulation according to aspect 15, wherein the cannabinoid and the moisture adsorbent are present in a weight ratio cannabinoid : moisture adsorbent of 0.14 - 0.44, preferably 0.19 - 0.39, in particular 0.24 - 0.34.
17. A formulation according to any of aspects 15 and 16, wherein the moisture adsorbent comprises a silicon dioxide.
18. A formulation according to any of aspects 1 to 17, wherein the solid dispersion is free or essentially free of triglycerides; and/or mono- and diglycerides; and/or fatty acids.
19. A formulation according to any of aspects 1 to 18, wherein the cannabinoid is cannabidiol (2-[(1R,6R)-3-methyl-6-(1-methylethenyl)-2-cyclohexen-1-yl]-5-pentyl-1,3-benzenediol).
20. A formulation according to any of aspects 1 to 19, consisting of a cannabinoid, an amphiphilic block copolymer as a solubilizer, a water-soluble film former, an antioxidant, a diluent and a moisture adsorbent and not more than 10 % by weight of other components, preferably not more than 5 % by weight of other components, in particular not more than 2 % of other components, relative to all components of the formulation.
21. A formulation according to aspect 20, wherein the cannabinoid is cannabidiol; wherein the amphiphilic block copolymer is a poloxamer, in particular poloxamer 188; wherein the water soluble film former is polyvinylpyrrolidone, in particular PVP K-30.
22. A formulation according to aspect 21, wherein the components are present in a weight ratio cannabinoid: the amphiphilic block copolymer : polyvinylpyrrolidone 1: 0.21 - 0.31 : 0.13 - 0.23.
23. A formulation according to any of aspects 1 to 22, wherein a micellar solution is formed upon combination of the formulation with an aqueous medium.
24. A formulation according to any of aspects 1 to 23, wherein the formulation, when subjected to an in vitro dissolution test in 0.1N HCl + 2 % CTAB following the USP paddle method, releases at least 75 wt% of the cannabinoid within 60 minutes, in preferably at least 90 wt% within 60 minutes.
25. A formulation according to any of aspects 1 to 24, wherein the formulation, when subjected to an in vitro dissolution test in 0.1N HCl + 2% CTAB following the USP paddle method, releases at least 75 wt% of the cannabinoid within 45 minutes, preferably at least 85 wt% within 45 minutes.
26. A formulation according to any of aspects 1 to 25, wherein a bioavailability is achieved which is not inferior compared to that of an oily cannabinoid solution 100 mg/ml as a reference product.
27. A formulation according to aspect 26, wherein the bioavailability is expressed by the AUC over an interval of 24 hours.
28. A formulation according to aspect 27, wherein the AUC is determined after a light meal of 350 - 600 kcal taken within 30 min prior to each administration within the 24 hours interval of determining the AUC.
29. A method for preparing a formulation as defined in any of aspects 1 to 28, wherein the method comprises the following steps:
   (i) preparing a liquid composition comprising the cannabinoid, the amphiphilic block copolymer and a solvent capable of at least partially dissolving the cannabinoid and the amphiphilic block copolymer;
   (ii) introducing the liquid composition into a fluid bed granulator;
   (iii) removing solvent to obtain a solid dispersion in particulate form; and
   (iv) recovering the solid dispersion in particulate form from the fluid bed granulator.
30. A method according to aspect 29, wherein the liquid composition in addition comprises the water-soluble film former.
31. A method according to aspect 29 or 30, wherein the liquid composition is sprayed into a fluid bed granulator already containing solid particles.

### Examples

The invention is illustrated with the help of specific examples, without being restricted in any way thereby.

### Example 1

### Preparation of granules

Cannabidiol (CBD) granules containing 29.7 % w/w active ingredient are prepared according to the following batch formula:

| Constituent | Function | Quantity (g) | Percentage in the final granulate (%) |
|---|---|---|---|
| CBD | drug substance | 170.78 | 29.7 |
| Poloxamer 188 *(Kolliphot*^{®} *P 188)* | solubilizer | 44.28 | 7.7 |
| Ascorbyl palmitate | antioxidant | 2.30 | 0.4 |
| Microcrystalline Cellulose *(Avicel*^{®} *PH 101)* | diluent | 278.30 | 48.4 |
| Colloidal silicon dioxide *(Aerosil*^{®} *200)* | moisture adsorbent | 46.00 | 8.0 |
| Polyvinylpyrrolidone *(Kollidon*^{®} *30)* | binder, solubilizer | 30.48 | 5.3 |
| Silicon Dioxide *(Syloid*^{®} *244 FP Silica)* | moisture adsorbent, glidant | 2.88 | 0.5 |
| Ethanol 96% v/v (a) | granulation liquid | 1150.00 | - |
| **Total theoretical weight** | | 575.02 | 100.0 |

In the first processing step, CBD and the pharmaceutical excipients poloxamer 188, ascorbyl palmitate, microcrystalline cellulose, silicon dioxide, colloidal silicon dioxide and polyvinylpyrrolidone are granulated.

For granulation, the fluid bed granulation technology is used.

The drug substance cannabidiol and the pharmaceutical excipients poloxamer 188, ascorbyl palmitate and polyvinylpyrrolidone are dissolved in ethanol 96% v/v. Silicon dioxide (Syloid^{®} 244 FP) is dispersed in the solution.

Microcrystalline cellulose and colloidal silicon dioxide (Aerosil^{®} 200) are charged into the fluid bed granulator and granulated with the described solution. The granules are discharged and sieved.

The volatile component ethanol 96% v/v is removed from the granules during the drying phase in the fluid bed dryer. The inlet air temperature is 45 ± 10°C, the product temperature 30 - 35°C.

The granules are dried up to a reference value for the loss on drying (LOD) percentage of not more than 2.0%.

### Dosage Form

Cannabidiol granules containing 29.7% w/w cannabidiol are filled in HDPE bottles to provide a total dose of 1500 mg Cannabidiol. The granulate is administered with 240 ml tap water (room temperature) in total. The granulate is firstly dispersed in 100 ml water. The remaining amount of water is used to rinse the container twice.

### Stability of the Cannabidiol Granulate

Samples are stored under accelerated conditions (40°C/75%), under intermediate conditions (30°C/65% rh) and under long-term conditions (25°C/60% rh).

Under storage at accelerated storage conditions the appearance discolored from white to yellowish after one months and to yellow after two months. The color changes only slightly to off-white at long-term conditions after three months and at intermediate conditions after four months.

The dissolution decreases slightly for storage at accelerated conditions after three months but is still well within specification. The dissolution remains unchanged after three months at long-term and after four months at intermediate conditions.

A decrease of assay of about 6 % at accelerated conditions after three months is observed, but the product is still within the shelf-life specification. At intermediate and long-term conditions no significant decrease of assay is observable after four months and three months, respectively.

### Stability of an Aqueous Dispersion

The chemical stability of an aqueous dispersion containing 1500 mg of cannabidiol was tested in a holding time study. For this purpose, about 5 g of a development batch (formulation without Aerosil 200) was dispersed in 240 ml water and stirred at ambient temperature. The impurity profile was monitored for 2 hours.

The impurity profile remains unchanged for the examined time period of two hours. Thus, the dispersion of the product in water for administration will be stable for a time period required for administration.

### CBD Release

Release is tested according to EP 2.9.3 / USP <711>. A paddle dissolution apparatus is used. Dissolution testing is performed at a standard temperature of 37°C ± 0,5°C and a stirrer speed of 100 rpm.

Complete release is observed in 0.1 M HCl + 2% cetyltrimethylammonium-bromide (CTAB) after 45 min.

### Bioavailability and Pharmacokinetic Properties

Bioavailability and pharmacokinetic properties of cannabidiol when administered orally in the form of the above described CBD granules containing 29.7% w/w cannabidiol was assessed in a clinical study.

The study involved a comparison with a reference product, an oily cannabidiol solution (DAC C-052 "Cannabidiol" / NRF 22.10 "Olige Cannabidiol-Lösung 100 mg/ml" ("Oily cannabidiol solution 100 mg/ml")) and was set up as an open-label, randomised (order of treatments), balanced, multiple dose trial was performed in a 2-period, 2-sequence-crossover design with direct switch-over.

Eighteen (18) healthy subjects of both sexes were randomised. The inclusion criteria involved a body-mass index (BMI) ≥18.5 kg/m² and ≤ 30.0 kg/m².

The investigational medicinal products were administered after a light meal as single oral doses of 1500 mg cannabidiol (i.e., 5.051 g granules dispersed in water or 15 ml solution) twice daily (i.e., every 12 h) over 7 consecutive days.

Steady-state characterisation was performed over 2 dosing intervals. Blood sampling was performed after the 13^{th} administration over 24 h, thereby including the 14^{th} administration, in order to characterise pharmacokinetic parameters after multiple dosing over a whole day interval. Additionally, a pre-dose sample was taken prior to the 1^{st} administration in period I and through values were obtained prior to the morning and evening administrations on study days 5 and 6 as well as the morning administration on study day 7 in order to characterise steady-state built-up phase.

Intake of food and beverages was standardised as follows.

In the morning and evening of study days 1 to 6 (ambulatory visits or during hospitalisation, respectively) subjects received a snack of approximately 350 - 600 kcal within 30 min prior to administration of the investigational medicinal products. The content / composition of the snack was not standardised except for the range of calorie content.

Further meals on study day 6 were served at appropriate times. Dinner (light meal) was finished at least 8 h prior to the intended time of start of breakfast (light meal) in the morning of study day 7. Fluid intake of water was ad libitum.

During the PK profiling day (study day 7), subjects received standardised meals at the following time points:
- light meal starting 30 min prior to the morning administration of study day 7;
- 4 h, and 8 h after the preceding administration;
- light meal starting 30 min prior to the evening administration of study day 7.

The light meal provided consisted of a three-seed-roll, a slice of cheese, two slices of chicken breast filet, a piece of butter, honey, one tomato, and one banana with a total caloric content of 543 kcal.

Fluid intake was standardised on the PK profiling day (study day 7) in the following way:
- From 0 h until 1 h p.a. no fluid intake.
- From 1 h until 10 h after the morning administration 150 ml of non-carbonated water (room temperature) were given every hour, i.e., 150 ml were given 10 times.
- From 10 h to 11 h p.a. intake of non-carbonated water was ad libitum. From 11 h to 12 h p.a. (i.e., within 1 h prior to the next administration) no fluid intake.
- From 1 h until 4 h after the evening administration 150 ml of non-carbonated water (room temperature) were given every hour, i.e., 150 ml were given 4 times.

Afterwards intake of non-carbonated water was ad libitum.

For pharmacokinetic determination blood samples were withdrawn at the following timepoints (1 pre-dose sample + 2 x 31 samples = 63 samples per subject):
- pre-dose sample: within 1.0 h prior to 1^{st} administration of the investigational medicinal product in period I
- trough samples: within 5 min prior to the 9^{th}, 10^{th}, 11^{th}, 12^{th}, and 13^{th} administration
- post-dose samples after 13th administration (including the 14^{th} administration): 0.5 h, 1 h, 1.5 h, 2 h, 2.5 h, 3 h, 3.5 h, 4 h, 4.5 h, 5 h, 6 h, 8 h, 12 h, 12.5 h, 13 h, 13.5 h, 14 h, 14.5 h, 15 h, 15.5 h, 16 h, 16.5 h, 17 h, 18 h, 20 h and 24 h after the 13th administration

Cannabidiol (CBD) and its metabolite 7-hydoxy cannabidiol (7-OH-CBD) in plasma samples were analysed by use of a validated LC-MS/MS; LLOQ for CBD was 5.00 ng/ml and for 7-OH-CBD was 1.000 ng/ml.

The following table (Table 1) shows pharmacokinetic parameters of cannabidiol after oral multiple dose administration of the cannabidiol granules twice daily over 7 consecutive days after a light meal (1,500 mg CBD per single dose; 21,000 mg CBD per treatment) obtained over 24 h for the last 2 dosing intervals after the 13^{th} and 14^{th} administration (from 144 hours to 168 hours of the administration period)

| | AUC_{144-168,ss} (h*ng/mL) | C_{max,144-168,ss} (ng/mL) | C_{min,144-168,ss} (ng/mL) | t_{max,144-168,ss} (h) | t_{min,144-168,ss} (h) | PTF %,₁₄₄₋₁₆₈ (%) | Cav,₁₄₄₋₁₆₈ (ng/mL) |
|---|---|---|---|---|---|---|---|
| N | 14 | 14 | 14 | 14 | 14 | 14 | 14 |
| Mean | 8970 | 1050 | 113 | 7.64 | 7.09 | 249.26 | 374 |
| SD | 2910 | 405 | 37.0 | 6.00 | 6.23 | 56.83 | 121 |
| Min | 2730 | 332 | 53.9 | 2.00 | 0.00 | 172.00 | 114 |
| Median | 9170 | 1060 | 113 | 5.00 | 11.9 | 241.84 | 382 |
| Max | 13500 | 1760 | 177 | 17.0 | 13.0 | 364.35 | 563 |
| Geometric Mean | 8370 | 963 | 107 | 5.80 | | 243.50 | 349 |
| CV% Geometric Mean | 44.44 | 49.62 | 37.14 | 89.49 | | 22.64 | 44.44 |

The above table shows that a mean steady state AUC over 24 hours after repeated administration twice daily of 1500 mg cannabidiol granules according to the present invention is about 9000 (h*ng/mL), such as 9000 +/- 3000 (h*ng/mL).

The mean steady state peak trough fluctuation (PTF) over 24 hours after repeated administration twice daily of 1500 mg, which can be determined as PTF = {[C_{max,144-168,ss} - C_{min,144-168,ss}]/C_{av,144-168,ss} } x 100, is about 250 %, such as 250 +/- 60 %.

The following table (Table 2) shows pharmacokinetic parameters of cannabidiol after oral multiple dose administration of the reference product twice daily over 7 consecutive days after a light meal (1,500 mg CBD per single dose; 21,000 mg CBD per treatment) obtained over 24 h for the last 2 dosing intervals after the 13^{th} and 14^{th} administration (from 144 hours to 168 hours of the administration period)

| | AUC_{144-168,ss} (h*ng/mL) | C_{max,144-168,ss} (ng/mL) | C_{min,144-168,ss} (ng/mL) | t_{max,144-168,ss} (h) | t_{min, 144-168,ss} (h) | PTF %,₁₄₄₋₁₆₈ (%) | C_{av,144-168} (ng/mL) |
|---|---|---|---|---|---|---|---|
| N | 14 | 14 | 14 | 14 | 14 | 14 | 14 |
| Mean | 9360 | 1210 | 113 | 11.3 | 5.08 | 265.01 | 390 |
| SD | 4140 | 745 | 42.1 | 6.40 | 6.32 | 84.03 | 172 |
| Min | 1400 | 130 | 25.7 | 3.00 | 0.00 | 155.10 | 58.5 |
| Median | 8840 | 960 | 123 | 15.0 | 1.08 | 245.22 | 369 |
| Max | 15700 | 3090 | 170 | 18.0 | 14.0 | 446.08 | 656 |
| Geometric Mean | 8170 | 976 | 102 | 9.20 | | 254.14 | 341 |
| CV% Geometric Mean | 69.24 | 88.30 | 54.26 | 81.28 | | 30.04 | 69.24 |

The above data indicate that the solid formulations of the present invention achieve a bioavailability, expressed by the AUC, which is not inferior to the reference product.

By the term "not inferior to the reference product" is meant that the bioavailability is not statistically significantly lower.

Similar considerations apply with respect to the PTF observed for the solid formulations of the present invention, which is not statistically significantly higher than that observed for the reference product.

These observations in particular apply in case of administering the medicinal products after a light meal as described above.

The following table (Table 3) shows pharmacokinetic parameters of 7-hydoxy cannabidiol after oral multiple dose administration of the cannabidiol granules twice daily over 7 consecutive days after a light meal (1,500 mg CBD per single dose; 21,000 mg CBD per treatment) obtained over 24 h for the last 2 dosing intervals after the 13^{th} and 14^{th} administration (from 144 hours to 168 hours of the administration period)

| | AUC_{144-168,ss} (h*ng/mL) | C_{max,144-168,ss} (ng/mL) | C_{min,144-168,ss} (ng/mL) | t_{max,144-168,ss} (h) | t_{min,144-168,ss} (h) | PTF %_{,144-168} (%) | C_{av, 144-168} (ng/mL) |
|---|---|---|---|---|---|---|---|
| N | 14 | 14 | 14 | 14 | 14 | 14 | 14 |
| Mean | 2940 | 264 | 47.1 | 4.97 | 9.89 | 180.70 | 122 |
| SD | 1120 | 100 | 16.8 | 3.88 | 5.10 | 41.16 | 46.7 |
| Min | 1580 | 169 | 24.5 | 2.00 | 0.00 | 107.98 | 65.8 |
| Median | 2690 | 245 | 44.4 | 4.50 | 12.5 | 187.60 | 112 |
| Max | 5450 | 498 | 84.4 | 18.0 | 12.6 | 253.69 | 227 |
| Geometric Mean | 2780 | 250 | 44.6 | 4.26 | | 175.97 | 116 |
| CV% Geometric Mean | 35.17 | 33.78 | 35.12 | 54.24 | | 24.97 | 35.17 |

The following table (Table 4) shows pharmacokinetic parameters of 7-hydoxy cannabidiol after oral multiple dose administration of the reference product twice daily over 7 consecutive days after a light meal (1,500 mg CBD per single dose; 21,000 mg CBD per treatment) obtained over 24 h for the last 2 dosing intervals after the 13^{th} and 14^{th} administration (from 144 hours to 168 hours of the administration period)

| | AUC_{144-168,ss} (h*ng/mL) | C_{max,144-168,ss} (ng/mL) | C_{min,144-168,ss} (ng/mL) | t_{max,144-168,ss} (h) | t_{min,144-168,ss} (h) | PTF %_{,144-168} (%) | C_{av, 144-168} (ng/mL) |
|---|---|---|---|---|---|---|---|
| N | 14 | 14 | 14 | 14 | 14 | 14 | 14 |
| Mean | 2830 | 292 | 43.3 | 11.6 | 10.6 | 211.78 | 118 |
| SD | 1140 | 124 | 14.6 | 6.61 | 5.34 | 47.44 | 47.4 |
| Min | 1080 | 116 | 16.7 | 3.00 | 0.500 | 135.06 | 44.9 |
| Median | 2680 | 268 | 43.9 | 15.5 | 13.0 | 210.64 | 112 |
| Max | 4800 | 512 | 64.2 | 20.0 | 14.5 | 304.02 | 200 |
| Geometric Mean | 2610 | 268 | 40.6 | 9.40 | 7.11 | 206.86 | 109 |
| CV% Geometric Mean | 45.08 | 46.29 | 41.77 | 82.08 | 198.99 | 22.92 | 45.08 |

### Example 2

Additional granulates were prepared following the method outlined in Example 1. Information on the composition is contained in the following table.

| | Batch | | | | | |
|---|---|---|---|---|---|---|
| | 200619 | 210076 | 210079 | 210112 | 210113 | 210114 |
| **Composition (wt %)** | | | | | | |
| Canapure PH | 29.7 | 29.7 | 29.7 | 29.7 | 30.4 | 31.6 |
| Avicel PH 101 | 48.4 | 48.4 | 49.7 | - | - | 46.0 |
| Pearlitol 160 C | - | - | - | 48.4 | 47.3 | - |
| Aerosil 200 | 8.0 | - | - | - | - | - |
| Syloid 244 FP | 0.5 | 8.5 | 12.5 | 8.5 | 8.5 | 8.1 |
| Kolliphor P 188 | 7.7 | 7.7 | 7.7 | 7.7 | 7.9 | 8.2 |
| PVP K-30 | 5.3 | 5.3 | - | 5.3 | 5.4 | 5.6 |
| Ascorbylpalmitat | 0.4 | 0.4 | 0.4 | 0.4 | 0.4 | 0.4 |
| | | | | | | |
| **Release after 45 min (wt %)** | 98.7 | 96.22 | 99.64 | 86.78 | 91.67 | 96.64 |

Pearlitol 160 C is a crystalline D mannitol powder having average mean particle diameter of 160 µm.

Release was determined using an in vitro dissolution method (1000 mL 0.1 M HCl + 2% (w/v) CTAB).

## Claims

1. Pharmaceutical formulation in the form of a solid dispersion, wherein the solid dispersion comprises in admixture a cannabinoid, an amphiphilic block copolymer as a solubilizer, a water-soluble film former and an antioxidant.

2. A formulation according to claim 1, wherein the antioxidant is selected from ascorbyl palmitate, alpha-tocopherol, butylhydroxytoluol (BHT, E321), butylhydroxyanisol (BHA, E320), ascorbic acid, and ethylenediaminetetraacetic acid (EDTA) sodium.

3. A formulation according to any of claims 1 or 2, wherein the antioxidant is ascorbyl palmitate.

4. A formulation according to any of claims 1 to 3, wherein the antioxidant is used in an amount of 0.5 to 2.5 wt%, preferably of 0.8 to 2 wt%, in particular 1.0 to 1.8 wt%, relative to the amount of the cannabinoid.

5. A formulation according to any of claims 1 to 4, wherein the cannabinoid and the amphiphilic block copolymer are present in a weight ratio cannabinoid: amphiphilic block copolymer of 1 : 0.11 - 0.41, preferably 1 : 0.16 - 0.36, more preferably 1: 0.21 - 0.31.

6. A formulation according to any of claims 1 to 5, wherein the cannabinoid and the water soluble film former are present in a weight ratio cannabinoid : water soluble film former of 1 : 0.03 - 0.33, preferably 1 : 0.08 - 0.28, more preferably 1 : 0.13 - 0.23.

7. A formulation according to any of claims 1 to 6, wherein the solid dispersion comprises a moisture adsorbent.

8. A formulation according to claim 7, wherein the cannabinoid and the moisture adsorbent are present in a weight ratio cannabinoid : moisture adsorbent of 0.14 - 0.44, preferably 0.19 - 0.39, in particular 0.24 - 0.34.

9. A formulation according to any of claims 1 to 8, wherein the cannabinoid is cannabidiol (2-[(1R,6R)-3-methyl-6-(1-methylethenyl)-2-cyclohexen-1-yl]-5-pentyl-1,3-benzenediol).

10. A formulation according to any of claims 1 to 8, wherein the cannabinoid is cannabidiol; wherein the amphiphilic block copolymer is a poloxamer, in particular poloxamer 188; wherein the water soluble film former is polyvinylpyrrolidone, in particular PVP K-30.

11. A formulation according to any of claims 1 to 10, wherein the color of the formulation remains stable or changes only slightly to off-white upon storage for three months, preferably for six months and in particular for 12 months under long-term conditions (25°C/60% rh).

12. A method for preparing a pharmaceutical formulation in the form of a solid dispersion, wherein the method comprises the following steps:
(i) preparing a liquid composition comprising the cannabinoid, the amphiphilic block copolymer and a solvent capable of at least partially dissolving the cannabinoid and the amphiphilic block copolymer;
(ii) introducing the liquid composition into a fluid bed granulator;
(iii) removing solvent to obtain a solid dispersion in particulate form; and
(iv) recovering the solid dispersion in particulate form from the fluid bed granulator.

13. A method according to claim 12, wherein the liquid composition in addition comprises the water-soluble film former.

14. A method according to any of claims **11** to 13, wherein the liquid composition also comprises an antioxidant in at least partially dissolved form.

15. A method according to any of claims **11** to 14, wherein the liquid composition is sprayed into a fluid bed granulator already containing solid particles.
